# EUROPEAN PATENT SPECIFICATION

(11) **EP 3 297 724 B1**
(45) Date of publication and mention of the grant of the patent: **16.08.2023**
(21) Application number: 16745197.0
(22) Date of filing: 19.05.2016
(51) Int. Cl.: A61N 1/40, A61N 1/44

(54) **ELECTRODE ARRANGEMENT FOR WOUND TREATMENT**
ELEKTRODENANORDNUNG FÜR WUNDBEHANDUNG
DISPOSITION DES ÉLECTRODES POUR TRAITMENT DES PLAIES

(30) Priority: 19.05.2015 US 201562163578 P
(43) Date of publication of application: 28.03.2018
(73) Proprietor: PlasmaCure B.V., 6534 AT Nijmegen (NL)
(72) Inventor: PEMEN, August Johannes Marie, 6534 AT Nijmegen (NL); ZEPER, Wouter Bastiaan, 6534 AT Nijmegen (NL)
(74) Representative: V.O.
(86) International application number: PCT/NL2016/050359
(87) International publication number: WO 2016/186501

(56) References cited:
- WO-A1-2013/040542
- US-A1- 2003 168 009
- US-A1- 2011 022 043
- US-B2- 9 005 188

## Description

### FIELD OF THE INVENTION

This invention relates to an electrode arrangement for wound treatment of an irregularly three-dimensionally shaped surface of an electrically conducting body, which surface is used as a counter electrode. In particular, the invention relates to devices that can be applied e.g. for preventing diabetic foot complications.

### BACKGROUND OF THE INVENTION

Cold plasmas have considerable potential for skin conditioning, disinfection of skin and wound healing. However, available plasma sources lack the possibility to treat larger areas, to control plasma properties and/or the possibility to adapt the shape of the plasma to the shape of the object to be treated (e.g. a foot). This invention focuses on: a cold plasma device, which can treat a larger area.
- Cold plasmas allow efficient, contact-free and painless disinfection, even in microscopic openings, without damaging healthy tissue.
- The bacterial response to plasma application is almost instantaneous (few seconds).
- The plasma is directly in contact with the skin which may result in fast disinfection of the skin.
- When operated in air a high production of NO radicals may be expected. The production of NO might have a positive effect on the microcirculation of blood in the skin, which is very important in the case of diabetic foot.
- A considerable, transient electric field will be present in/near the skin (several kV/cm). Such electrical fields result in faster proliferation of skin cells. Also a positive effect on blood circulation might occur due to electroporation of skin.
- Provides a microbial defense system for plasma application (and hence resistance build- up).
- Plasmas can stimulate human cells without damage. Clinical trials confirm the efficacy and tolerability of plasma in treating infected chronic wounds.
- Plasmas could contribute to a better skin condition and a higher ability for wound healing due to an improved microcirculation.
- Plasma device according to this invention support preventing serious foot complications at an early stage, and contributes to skin improvement, wound prevention and healing.
- Nowadays, the only preventive actions that people with diabetes mellitus can take at home, is daily inspection and daily cleaning of the feet. However, this is often not practical for high-risk patients due to poor eyesight, obesity and stiff joints. World- wide there are no homecare devices available.
- The invention provides an easy to use, fool-proof homecare device that solves this problem.
- Because of the costs involved it is crucial that patients treat their feet every day. However, patients with diabetic foot complications are relatively old and tend to forget.

The flexible plasma device is a platform technology with a number of interesting applications and market possibilities to improve skin conditions, prevent ulcerations and accelerate healing of the diabetic foot. The plasma can easily be delivered to the skin of a patient, e.g. in the form of a plasma plaster. The skin will be temporary exposed to the plasma to disinfect the skin and to improve cell proliferation and microcirculation of the blood. Typically, one-minute plasma treatment will reduce the bacterial load on the foot with up to a factor of one million, without negatively affecting the skin. Such a treatment should be continued on a once/twice per day basis until the threat of infection has been overcome.

This technology offers perspectives for medical treatments and prevention measures. In dermatology, new opportunities are being opened for wound healing, tissue regeneration, therapy of skin infections, and probably many more applications. Also, plasmas may effectively kill skin-cancer cells. A few examples:
- Skin diseases: Most dermatological problems are associated with bacterial or fungal infections. Plasmas may help to reduce complications due to bacteria and fungi, and may even treat the diseases themselves.
- Chronic wounds and inflammations: Plasmas may very well assist in controlling the consequences of chronic inflammation associated with these diseases by eliminating bacterial and fungal infections, which results in a drastic improvement of the quality of life.
- Hospital hygiene: The growth of resistant bacteria (e.g. MRSA) poses a big problem in hospitals. Plasma devices can sterilize or disinfect both medical tools and hands (e.g. of surgeons).
- Antifungal treatment: It has been shown that plasmas can be employed efficiently to combat fungal diseases.

From US9005188 and EP2670477 flexible electrodes are known with a structured surface of a plurality of spaced apart projections from the surface to form air-guiding areas where the plasma is generated.

### SUMMARY OF THE INVENTION

In summary, embodiments of the invention pertain to: an electrode arrangement according to the features of claim 1.

The present invention, rather than having a flexible electrode with projections as known from US9005188, provides a compartmented structure such that upon contact with the skin a number of closed compartments is realized where the plasma is generated. The advantage of having closed compartments, rather than the open air guiding areas, is that the main chemical components generated by the plasma (e.g. ozone) is/are fixed in a closed environment. This prevents release of ozone to the environment and increases the effectiveness of the plasma generated.

### BRIEF DESCRIPTION OF THE DRAWINGS

Figure 1 shows a schematic perspective view of a first embodiment of the cold plasma device;
Figure 2 shows the device of Figure 1 in schematic cross sectional view;
Figure 3A and B show edge details of the cross sectional view in Figure 2;
Figure 4 show various embodiments of the planar electrode of the cold plasma device of the previous Figures;
Figure 5 shows another embodiment of the cold plasma device;
Figure 6 provides a schematic cross sectional view of the embodiment of Figure 5.
Figure 7 shows a further embodiment of the cold plasma device;
Figure 8 shows yet another embodiment of the cold plasma device;
Figure 9 shows an embodiment of the cold plasma device in use.

### DETAILED DESCRIPTION

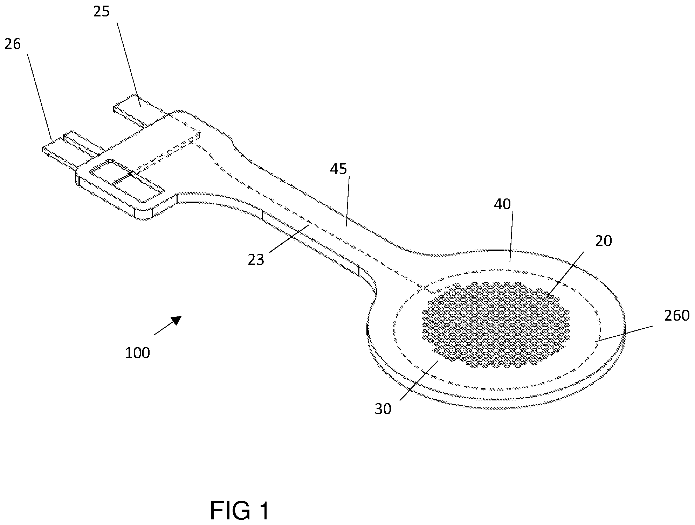

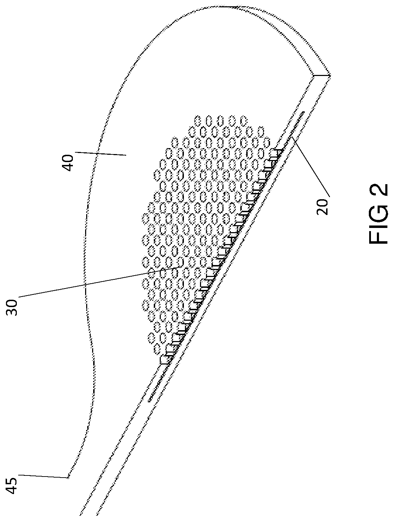

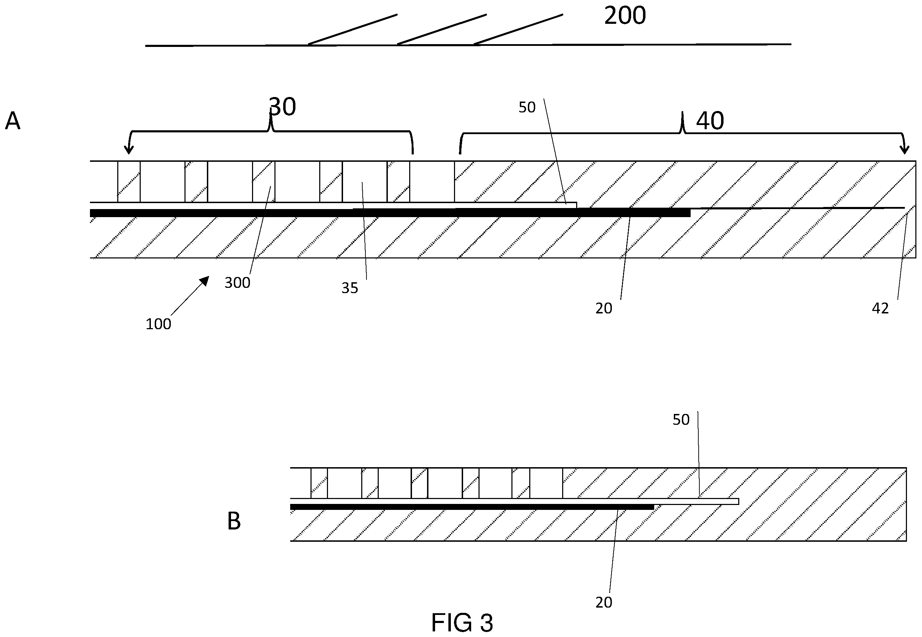

Figure 1 shows a schematic perspective view of a prototype of the cold plasma device. Figure 2 shows the device of Figure 1 in schematic cross sectional view. Figure 3 shows an edge detail of the cross sectional view in Figure 2. The plasma device 100 provides a dielectric barrier discharge (DBD) technique for plasma plaster development. The DBD concept will be briefly explained here with reference to Figure 3. A DBD type of plasma source has a planar electrode 20 that is covered with a dielectric foil or film 50. A gas (or air) gap is present in a compartment 30 formed between an object to be treated (e.g. foot) being functioning as electrode 200 and dielectric 50 with a dielectric constant e.g. larger than 2. For ease of understanding in Figure 3A, object to be treated 200 is shown apart from device 100, but in use, edge 40 firmly contacts object 200.The electrode 20 is powered by pulsed or AC high-voltage. Due to the dielectric 50, the electric field is mainly present in the air gap 35 formed by protrusions 300 in the structured surface 30. The protrusions 300 ensure a minimum distance of the electrode 20 to the object 200 e.g. larger than 1mm. If the electric field is high enough (>30 kV/cm) and if the thickness of the air gap is rather constant, homogeneous cold plasma is created in the air gap to the object to be treated (e.g. the skin of a foot). Dielectric and protrusions have a high dielectric strength, e.g. > 180 kV/mm.

A DBD cold plasma device can treat large areas; the dimensions of the DBD can be chosen over wide margins. Instead of allowing for airflow between the cold plasma device and the skin, discrete compartments 30 are formed that will contain some air, but these need not be connected to each other, they are isolated from each other, and also isolated to the surroundings by a closed edge.

The advantage is that the reactive gases that we will generate during operation of the cold plasma, gases like ozone cannot escape. This has the advantage that the device is more efficient: all reactive specimens are available to kill pathogens, and that the release of any toxic gases like ozone will be minimized.

Further to this invention, the compartments can have any shape as long as they are separated from each other, and also they may vary in size and shape, even within one device. Also, each device will have a closed edge in order to prevent release of any gases to the surroundings.

In addition: only with a "closed system" the embodiments as further described will be possible. These inventions include gas control, gas-recipes from within the cold plasma device.

The device can have any shape, round square, irregularly shaped, and have an edge (typically 1 cm) as described earlier.

Accordingly an electrode arrangement 100 is shown for a dielectric barrier discharge plasma treatment of an irregularly three-dimensionally shaped surface of an electrically conducting body. The body is typically a human body part, such as a foot, heel, toe, finger or any other diseased skin part, which surface is used as a counter electrode. The arrangement has a first planar electrode 20 to be coupled to a high voltage source a dielectric 50 (see Figure 3) which is formed by a flexible material in such a way that the dielectric 50 shields the first planar electrode from the surface to be treated; a spacer structure 30 defining a structured surface on a side of said arrangement 100 facing a surface 200 to be treated, such that the structured surface forms one or more spaced compartments 30 that are isolated by an edge 40 from the surroundings in order to prevent airflow between the surroundings and the compartments 30.

The device 100 has a an electrode 20 that is fitted to the object to be treated 200 and brought in contact with the dielectric, in order to provide a substantially conformal compartment that follows the contours of the 3D shaped body for providing a homogenous microdischarged plasma. By an electrode 20 fitted to the object to be treated, the occurrence of saddle points or sharp folds prevents undesired local field strengths. It may be desirable to shape the electrode centrally to a concave or convex form of the surface to be treated, to optimize the local stretch of the electrode 20, so that the device optimally adapts to the object 200. A particularly suitable embodiment is provided wherein the first planar electrode is a mesh. In contrast to a metal foil, a mesh is suitably adaptable to the 3D shape, and will not rupture, crease or fold. The mesh may be contacted by a twisted pair lead 23, that connects to a high voltage clamp 25, and a ground electrode clamp 26 that connects to the second electrode respectively, said lead integrated in lead portion 45 integral to the edge 40. It was found that a mesh is stretchably deformable around three dimensional of an object to be treated, such as a heel, finger or toe, while still being able to provide a suitable homogenous plasma. It will be understood that 'stretchable' is to be understood in a conventional context known to the skilled person, i.e. in contrast to merely being flexible but non deformable in a planar length dimension, able to deform in a planar dimension typically more than 2-5% or even 10% of a length dimension. Furthermore, it can be seen that the structured surface comprises an edge portion 40 wherein the first planar electrode 20 extends into the edge portion 40. By extending the planar electrode 20 into the edge portion 40 a suitable solution may be provided for a problem of preventing break through near the edge portion of the electrode 20.

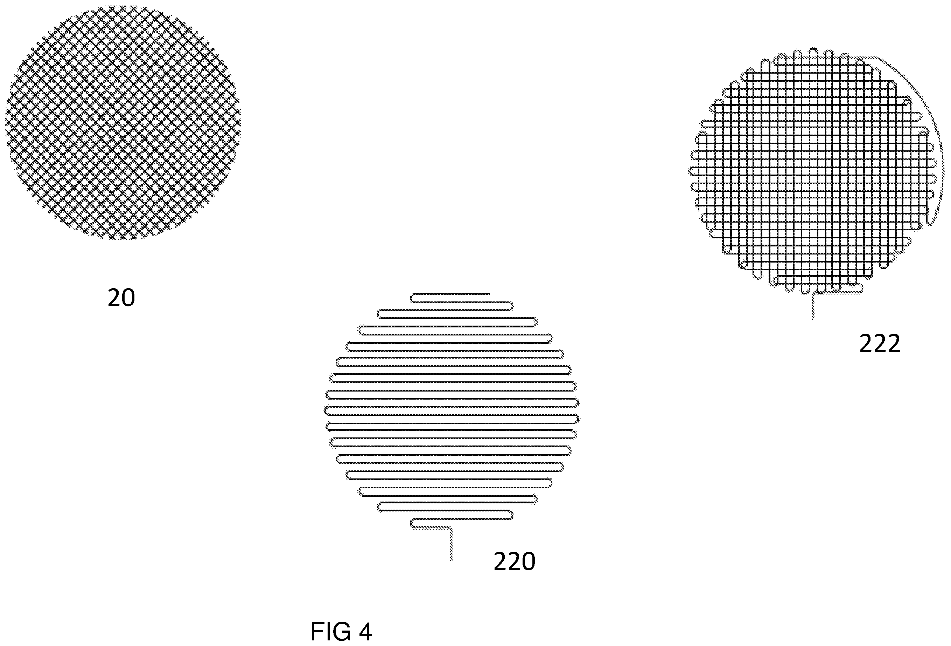

Figure 4 shows a variety of electrode structures 20, 220 and 222. It is shown that the mesh may be provided by woven conductive threads, typically metallic, with a weave that allows in plane deformations. In contrast to electrode 20, that has a weave with straight non-connected parallel wires, electrode structure 220 by forming the first electrode from a continuous conductive wire. In this embodiment, a weave from a single wire, or from wires with a non-intersecting meandering pattern, further prevents breakthrough problems. In another electrode weave 222, the wire is provided with a in intersecting meandering weave pattern.

Variations to such embodiments, one could have the following additions or changes:
- When the high voltage (HV) connection comes from the side which has the advantage of maximum flexibility, and makes it easier to make the device "see through". In such an embodiment, the first planar electrode 20 may be contacted with a twisted pair lead 23, that connects to high voltage clamp 25, the twisted pair preventing high field strengths outside the planar electrode 20. A ground electrode clamp 26 connects to the second electrode 260 respectively, said lead 23 integrated in a lead portion 45 integral to the edge portion 40.
- When the HV connection comes from the rear, has the advantage that the device will be more compact
- The planar electrode 20 is connected to contact 25 with slide contacts of a PCB connector (not trivial for HV connectors) for easy operation and for costs reduction of the disposable part
- The electrode arrangement is substantially transparent, so that an underlying body and the created plasma can be visually inspected. Specifically the dielectric material and the electrode are transparent so that it is possible to see the plasma in operation and to see the area under the plasma device (the wound). More specifically the isolating cover layer and spacer are provided from a single transparent flexible preform, for example, a single transparent isolator that hinges on an edge part 42 (see Figure 3).
- The driver circuit may comprise a planar electrode identification circuit. The connector has a sense (or identification) contact (not shown), so that the driving unit can detect:
   - what kind of cold plasma device is connected and adjust it settings for currents and HV
   - if a cold plasma is connected at all, for safety purposes
   - if a cold plasma device is correctly connected, for safety purposes
      This can for example be realized by integrating a matching impedance or impedance circuit to the connector so that the impedance is matched to identify the connector.
- The plasma device can be used at various levels in the medical care system: by the patient himself (e.g. as a homecare device), in primary health care (e.g. by a podiatrist or family doctor), in an outpatient clinic, or in a medical centre or hospital.

### Electrical aspects

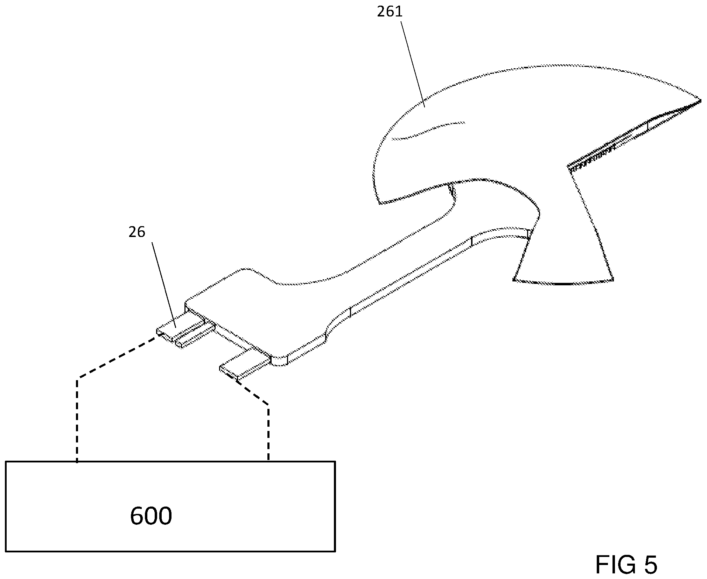

The plasma can be powered by repetitive, short high-voltage pulses (ns-µs duration, up to a few 100 kHz repetition rate). For example, in Figure 5 a driver circuit 600 is provided for driving the planar electrode, wherein the driver circuit drives the planar electrode in a pulsed voltage in a range of 3-8kV, in a range of 0.5-100kHz, and a pulse duration in a range of 1-150 micro second. This allows for a pulse rate that substantially provides a micro discharge wherein electrical current through the object to be treated (skin, human body) will only flow during the time that the plasma is on (which is typically equal to the HV pulse duration). In between the pulses, the plasma is not active, and no current flows through the skin. When the plasma would be powered by AC voltage, current flow happens during the entire AC cycle, while the plasma is active only during a small part of the AC cycle.

The pulsed operation of the plasma enables perfect control over the power of the plasma by means of the pulse repetition rate. In this way, the plasma power can be controlled and adjusted without affecting the plasma properties. When the plasma would be powered by AC voltage, controlling the power is only possible by means of the voltage magnitude, and thus affects the plasma properties.

Due to the pulsed operation, treatments can be performed at adjustable and controllable pulse sequences, duty cycles and bursts of pulses with varying duration. Pulse sequences can be optimized towards a specific application.

The grounded electrode 26 as shown in Figure 1 will enhance safety (prevents plasma generation and/or high-voltage to be present outside of the device), and also reduces electromagnetic emission of the device. This allows the device to be used without problems in any (electrical) environment.

More specifically, Figure 5 shows that a second planar electrode 261 completely covers said stretchable isolating cover layer. The second planar electrode 261 may be connected to the ground electrode 26. The second planar electrode 261 is furthermore connected to conductive ring electrode 260 that is provided in the edge portion. This conductive ring electrode may be formed with a conductive sticker edge that is attachable to the human skin. For a more precise attachment to 3D convex forms, such as a heel portion, it may be preferred that (parts of) the edge portion are non-stretchable. This may be important for the homogeneity of the electric field and corresponding plasma forming.

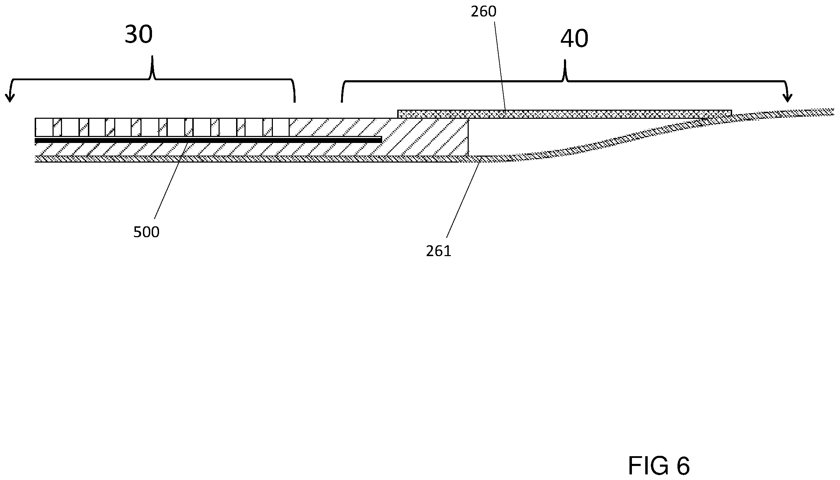

Figure 6 provides a schematic cross sectional view of the embodiment of Figure 5. For homogeneous plasma generation, a (rather) constant width of the gas gap (up to a few mm) is required. In this invention, a structured interface is provided (see figure 2) that provides a constant distance between the dielectric barrier and the object to be treated. This is an essential condition for the proper functioning of a DBD device. This can be even better accomplished by a dielectric 50 that is formed by a stack 500 of dielectric layers 50. A stack 500 of one or more thin foil dielectrics has a possibility to prevent any pinholes that would form an unshielded channel between the electrode and the grounded treatment surface, without any dielectric. This improves the homogeneity of the electric field and corresponding plasma forming.

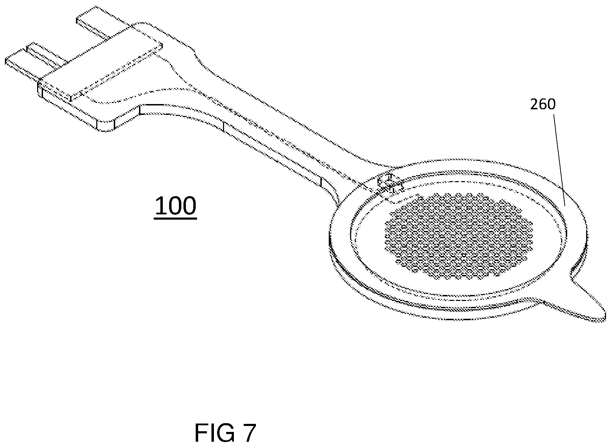

Figure 7 shows a further embodiment wherein the conductive ring 260 has an extending lip designed to slightly press into a skin portion for further ensuring grounding of the conductive ring 260.

### Further embodiments

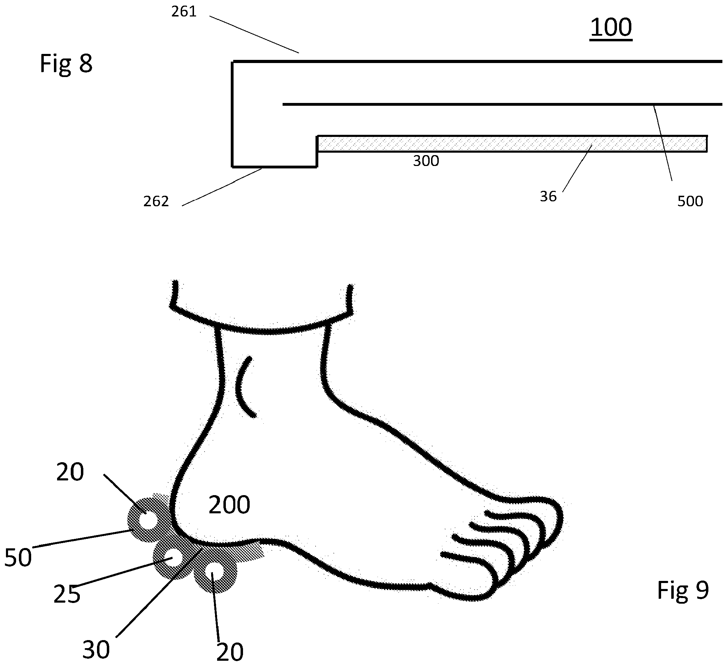

In a further embodiment, the device is provided with an interface 36 layer (See Figure 8) between the plasma electrode stack (including dielectric) 500 and the object to be treated so that the gas environment in which the plasma is generated can be tailored (and thus the plasma properties can be tailored) and that provides a constant distance to the object to be treated. Such a feature may solve a problem of gas control and may provide a specific form of plasma delivery in the form of tailor made 'plasma-recipes' via a gas composition that becomes available when the device is activated. The interface 36 may have a controlled composition of gases that in combination with HV driver settings will generate a tailor-made mixture of reactive gases. Also, this layer can act as absorber for e.g. unwanted O3. In short, the function of 36 is threefold:
1) Define the gas mixture (O2, N2, H2O, others) in the plasma, and steer the properties of the plasma,
2) Absorb unwanted gases, and
3) Set the distance between electrode and skin/human.

The properties of the cold plasma (such as the yields of reactive species and UV) depend on the type of gas that is present in the gas gap. The interface 36 between the plasma electrodes and the object to be treated allows buffering or storage of gas and the (triggered) slow release of gas during the time that the plasma is running. So the gas environment in which the plasma is generated can be tailored, without the need for gas bottles and associated tubing and flow control. Thus also the plasma properties can be tailored.

From an application point of view, the device can for example be integrated in a mold or plaster that fits to the part of the body that must be treated. Such assemblies can be made patient specific, and can be used during the entire treatment period of the patient.

The interface layer 36 may be constructed as an integral part of a patient specific plasma device, which simultaneously is designed to create an air gap. Alternatively the interface layer can also be a separate device that will be applied temporarily, e.g. in the form of a disposable. Before use, the disposable can be kept in a closed or sealed package or bag. It can be thrown away after use. Storing the disposable into a sealed package allows it to keep it sterile. So there is no need to clean the interface before use. A sealed package can also be filled with a preferred gas, thus supporting the buffering of gas in the interface.

Several options are available to construct the interface layer 36 of the plasma device 100. One option is to use a (thin) layer of silica aerogel material 36. Silicon aerogels find application in drug delivery and have good bio-compatibility. Aerogels have the required electrical properties (they also find application as high-voltage insulators), such as a dielectric constant between 2-4 and a high dielectric strength. Due to the high porosity (>85%) and homogeneous feature of aerogels, the generation of plasma is not hampered by such a layer. The high porosity and large surface area (>400 m2/gram) allows buffering and the slow release of gas. This has been demonstrated for hydrogen fuel storage and for drug delivery applications (where the drugs are in solution with CO2 gas). It is expected that gases such as nitrogen, helium, argon and air can be stored without any problem. The slow release of gas will start as soon as the device is taken out of its sealed package. The gas release rate can be controlled by the aerogel density and surface area. Gas release may also be triggered (e.g. by the intense electric field or the UV emission of the plasma). Accordingly the gas release rate can be affected by the plasma intensity.

Another option to construct the interface is to use a (thin) layer or a patch of polymer material. The polymer material contains gas reservoirs that are loaded with the preferred gas.

Plasma is generated into these gas reservoirs 300. Optionally, interface layer 36 provides a protrusion structure for forming these reservoirs and ensuring a minimal distance to the skin. The plasma products must be released to the object to be treated (skin). This can be done by for instance permeation and/or diffusion through the polymer membrane. Another way is to rupture the polymeric membrane by means of the plasma (e.g. by the UV or by the intense electric field).

A third option is to use polymeric gas dispenser materials. An example is AIBN that can be used to release N2. Another example is silver oxide that can be used to release O2. Such materials are used for instance as oxygen generator in airplanes or for very fast gas release in airbags. Generally, gas release is triggered thermally. Preferably gas release is triggered by the plasma (UV). Also more complex materials, such as zeolites, barium peroxide and azides can be used for gas dispenser applications. However, these materials have poor biocompatibility.

Important features of the device as in Figure 8 are:
- The gas environment in which the plasma is generated can be tailored, without the need for gas bottles and associated tubing and flow control. Thus also the plasma properties can be tailored towards optimal therapeutic use.
- The pulsed operation of the device allows perfect control over the power of the plasma in order to maximize the treatment efficiency while minimizing negative effects of a too high power. Treatment "protocols" can be developed and optimized (even patient specific).

Such an interface layer 36 can be provided by both a so called "dielectric barrier discharge" (DBD), or a surface dielectric barrier discharge (SDBD). By applying flexible materials (e.g. plastic or synthetic rubber foils/films, metallic foils, conductive fabrics) a flexible construction can be made, that allows to fit the shape of the plasma to the shape of the object to be treated see e.g. Figure 9, giving an impression how this device can be applied for diabetic foot treatment. E.g. the electrode form 20 is fitted to the object to be treated by closely following the contour of the heel 200. Such electrode form can be integrated into a silicon rubber mould 30 or into a plaster cast, which fits around a heel or toe. The electrode may e.g. have a dielectric coating 50 and can be of the stretchable mesh type as illustrated in Figure 4, e.g. woven from a single continuous wire 20. In some embodiments, the mesh electrode configuration 20 can be adapted to have a pair of high voltage wire electrodes 20 provided alternating parallel with grounded wiring 25, to create a surface dielectric barrier discharge between adjacent wire electrodes 20, 25.

To stimulate compliance, remote monitoring e.g. via Internet could be incorporated into the device.

## Claims

1. An electrode arrangement (100) for wound treatment of an irregularly three-dimensionally shaped surface of an electrically conducting body (200), which surface is used as a counter electrode, having
- a first planar electrode (20) to be coupled to a high voltage source which planar electrode (20) is covered with a dielectric foil or film (50)formed by a flexible material in such a way that the dielectric shields the first planar electrode (20) from the surface to be treated;
- a spacer (30) defining a structured surface on a side of said arrangement facing a surface to be treated, such that the structured surface forms one or more spaced compartments (30) that are isolated from each other by protrusions (300) ensuring a minimum distance of the electrode (20) to the object; and are isolated from the surrounding of the compartments by an edge (40) in order to prevent airflow between the surrounding and the compartments (30),
- said first planar electrode (20) being fitted to the object to be treated (200) and brought in contact with the dielectric, and
- an isolating cover layer covering the first planar electrode (20).

2. The electrode arrangement, according to claim 1, wherein the dielectric is formed by a stack (500) of dielectric layers.

3. The electrode arrangement, according to any of the previous claims, wherein the structured surface comprises an edge portion (40) wherein the first planar electrode (20) extends into the edge portion (40).

4. The electrode arrangement of claim 3, wherein the edge portion (40) is non-stretchable.

5. The electrode arrangement of any of the previous claims, wherein the first planar electrode (20) is a stretchable mesh.

6. The electrode arrangement of any of the previous claims, wherein the first planar electrode (20) is formed from a continuous conductive wire.

7. The electrode arrangement of any of the previous claims, wherein the first planar electrode (20) is formed from a conductive wire, that is coated with a dielectric.

8. The electrode arrangement of any of the previous claims, wherein the first planar electrode (20) is contacted with a twisted pair lead (23), that connects to a high voltage clamp, and a ground electrode clamp that connects to the second electrode (26) respectively, said lead integrated in a lead portion integral to the edge .

9. The electrode arrangement according to any of the previous claims, wherein the electrode arrangement is substantially transparent, so that an underlying body (200) and the created plasma can be visually inspected.

10. The electrode arrangement according to any of the preceding claims, wherein the isolating cover layer and spacer (30) are provided from a single transparent flexible preform.

11. The electrode arrangement according to any of the preceding claims, wherein the planar electrode is connected to a contact with slide contacts of a PCB connector.

12. The electrode arrangement according to any of the preceding claims further comprising a driver circuit for driving the planar electrode coupled to said high voltage source, wherein the driver circuit drives the planar electrode in a pulsed voltage in a range of 3-8kV, repetition rate in a range of 0.5-50kHz, and a pulse duration in a range of 0.1-100 micro second

13. The electrode arrangement according to claim 12 wherein the driver circuit comprises a planar electrode identification circuit.

14. The electrode arrangement according to any of the preceding claims wherein a further gas control structure is provided in the electrode arrangement, arranged to generate a plasma precursor gas.

15. The electrode arrangement of claim 14, wherein the gas control structure comprises a gas drain or gas absorbent.

## Patentansprüche

1. Elektrodenanordnung (100) zur Wundbehandlung einer unregelmäßig dreidimensional geformten Oberfläche eines elektrisch leitenden Körpers (200), wobei die Oberfläche als eine Gegenelektrode genutzt wird, umfassend
- eine erste planare Elektrode (20), die mit einer Hochspannungsquelle gekoppelt werden soll, wobei die planare Elektrode (20) mit einer dielektrischen Folie oder Film (50) bedeckt ist, die von einem flexiblen Material derart gebildet werden, dass das Dielektrikum die erste planare Elektrode (20) von der zu behandelnden Oberfläche abschirmt;
- ein Abstandhalter (30), der eine strukturierte Oberfläche auf einer Seite der Anordnung definiert, die einer zu behandelnden Oberfläche zugewandt ist, so dass die strukturierte Oberfläche eine oder mehrere beabstandete Abteile (30) bildet, die durch Vorsprünge (300) voneinander isoliert sind, die einen Mindestabstand der Elektrode (20) zum Objekt gewährleisten; und die von der Umgebung der Abteile durch eine Kante (40) isoliert sind, um einen Luftstrom zwischen der Umgebung und den Abteilen (30) zu verhindern,
- wobei die erste planare Elektrode (20) an dem zu behandelnden Objekt (200) angebracht und in Kontakt mit dem Dielektrikum gebracht wird, und
- eine isolierende Deckschicht, die die erste planare Elektrode (20) bedeckt.

2. Elektrodenanordnung nach Anspruch 1, wobei das Dielektrikum durch einen Stapel (500) dielektrischer Schichten gebildet ist.

3. Elektrodenanordnung nach einem der vorhergehenden Ansprüche, wobei die strukturierte Oberfläche einen Kantenabschnitt (40) umfasst, wobei sich die erste planare Elektrode (20) in den Kantenabschnitt (40) erstreckt.

4. Elektrodenanordnung nach Anspruch 3, wobei der Kantenabschnitt (40) nicht dehnbar ist.

5. Elektrodenanordnung nach einem der vorhergehenden Ansprüche, wobei die erste planare Elektrode (20) ein dehnbares Netz ist.

6. Elektrodenanordnung nach einem der vorhergehenden Ansprüche, wobei die erste planare Elektrode (20) aus einem kontinuierlich leitenden Draht gebildet ist.

7. Elektrodenanordnung nach einem der vorhergehenden Ansprüche, wobei die erste planare Elektrode (20) aus einem leitenden Draht gebildet ist, der mit einem Dielektrikum beschichtet ist.

8. Elektrodenanordnung nach einem der vorhergehenden Ansprüche, wobei die erste planare Elektrode (20) mit einer verdrillten Paarleitung (23) kontaktiert ist, die mit einer Hochspannungsklemme verbunden ist, bzw. einer Erdungselektrodenklemme, die mit der zweiten Elektrode (26) verbunden ist, wobei die Leitung in einen in die Kante integrierten Leitungsabschnitt integriert ist.

9. Elektrodenanordnung nach einem der vorhergehenden Ansprüche, wobei die Elektrodenanordnung im Wesentlichen durchsichtig ist, so dass ein darunterliegender Körper (200) und das erzeugte Plasma sichtgeprüft werden können.

10. Elektrodenanordnung nach einem der vorhergehenden Ansprüche, wobei die isolierende Deckschicht und der Abstandhalter (30) aus einer einzigen durchsichtigen biegsamen Vorform bereitgestellt sind.

11. Elektrodenanordnung nach einem der vorhergehenden Ansprüche, wobei die planare Elektrode mit einem Kontakt mit Gleitkontakten eines PCB Konnektors verbunden ist.

12. Elektrodenanordnung nach einem der vorhergehenden Ansprüche, ferner umfassend eine Treiberschaltung zum Antreiben der mit der Hochspannungsquelle gekoppelten planaren Elektrode, wobei die Treiberschaltung die planare Elektrode in einer gepulsten Spannung in einem Bereich von 3-8 kV, einer Wiederholungsrate in einem Bereich von 0,5-50 kHz und einer Pulsdauer in einem Bereich von 0,1-100 Mikrosekunden antreibt

13. Elektrodenanordnung nach Anspruch 12, wobei die Treiberschaltung eine planare Elektroden-Identifikationsschaltung aufweist.

14. Elektrodenanordnung nach einem der vorhergehenden Ansprüche, wobei in der Elektrodenanordnung eine weitere Gassteuerstruktur vorgesehen ist, die zur Erzeugung eines Plasmavorläufergases angeordnet ist.

15. Elektrodenanordnung nach Anspruch 14, wobei die Gassteuerstruktur einen Gasabfluss oder ein Gasabsorptionsmittel umfasst.

## Revendications

1. Agencement d'électrodes (100) pour le traitement de plaies d'une surface de forme tridimensionnelle irrégulière d'un corps électriquement conducteur (200), laquelle surface est utilisée comme contre-électrode, ayant
une première électrode plane (20) destinée à être couplée à un une source haute tension, l'électrode plane (20) étant recouverte d'une feuille ou d'un film diélectrique (50) formé d'un matériau flexible de telle sorte que le diélectrique protège la première électrode plane (20) de la surface à traiter ;
une entretoise (30) définissant une surface structurée sur un côté dudit agencement tourné vers une surface à traiter, de sorte que la surface structurée forme un ou plusieurs compartiments espacés (30) isolés les uns des autres par des protubérances (300) assurant une distance minimum de l'électrode (20) à l'objet ; et sont isolées de l'entourage des compartiments par un rebord (40) afin d'empêcher l'écoulement d'air entre l'entourage et les compartiments (30),
ladite première électrode plane (20) étant adaptée à l'objet à traiter (200) et amenée en contact avec le diélectrique, et
une couche de couverture isolante recouvrant la première électrode plane (20).

2. Agencement d'électrodes, selon la revendication 1, dans lequel le diélectrique est formé par un empilement (500) de couches diélectriques.

3. Agencement d'électrodes selon l'une quelconque des revendications précédentes, dans lequel la surface structurée comprend une partie de bord (40) dans laquelle la première électrode plane (20) s'étend dans la partie de bord (40).

4. Agencement d'électrodes selon la revendication 3, dans lequel la partie de bord (40) est non extensible.

5. Agencement d'électrodes selon l'une quelconque des revendications précédentes, dans lequel la première électrode plane (20) est une maille extensible.

6. Agencement d'électrodes selon l'une quelconque des revendications précédentes, dans lequel la première électrode plane (20) est formée à partir d'un fil conducteur continu.

7. Agencement d'électrodes selon l'une quelconque des revendications précédentes, dans lequel la première électrode plane (20) est formée à partir d'un fil conducteur, qui est revêtu d'un diélectrique.

8. Agencement d'électrodes selon l'une quelconque des revendications précédentes, dans lequel la première électrode plane (20) est mise en contact avec un conducteur à paire torsadée (23), qui se connecte à une pince haute tension, et une pince d'électrode de masse qui se connecte à la deuxième électrode (26) respectivement, ledit conducteur étant intégré dans une partie de conducteur solidaire du bord.

9. Agencement d'électrodes selon l'une quelconque des revendications précédentes, dans lequel l'agencement d'électrodes est sensiblement transparent, de sorte qu'un corps sous-jacent (200) et le plasma créé peuvent être inspectés visuellement.

10. Agencement d'électrodes selon l'une quelconque des revendications précédentes, dans lequel la couche de couverture isolante et l'entretoise (30) sont fournis à partir d'une seule préforme flexible transparente.

11. Agencement d'électrodes selon l'une quelconque des revendications précédentes, dans lequel l'électrode plane est connectée à un contact à contacts coulissants d'un connecteur PCB.

12. Agencement d'électrodes selon l'une quelconque des revendications précédentes comprenant en outre un circuit d'attaque pour piloter l'électrode plane couplée à ladite source haute tension, dans lequel le circuit d'attaque pilote l'électrode plane avec une tension pulsée dans une plage de 3 à 8 kV, une fréquence de répétition dans une plage de 0,5 à 50 kHz et une durée d'impulsion dans une plage de 0,1 à 100 microsecondes.

13. Agencement d'électrodes selon la revendication 12, dans lequel le circuit d'attaque comprend un circuit d'identification d'électrode plane.

14. Agencement d'électrodes selon l'une quelconque des revendications précédentes, dans lequel une autre structure de commande de gaz est prévue dans l'agencement d'électrodes, agencée pour générer un gaz précurseur de plasma.

15. Agencement d'électrodes selon la revendication 14, dans lequel la structure de commande de gaz comprend une évacuation de gaz ou un absorbant de gaz.
